**Europäisches Patentamt**

**European Patent Office**

**Office européen des brevets**

(19)

(11) Numéro de publication : **0 326 470 B1**

## (12) FASCICULE DE BREVET EUROPEEN

(45) Date de publication du fascicule du brevet :
16.10.91 Bulletin 91/42

(51) Int. Cl.⁵ : **C07C 69/716, C07C 67/313, C07C 67/30**

(21) Numéro de dépôt : **89400176.7**

(22) Date de dépôt : **20.01.89**

(54) Perfectionnement au procédé de fabrication de pyruvates d'alkyle.

(30) Priorité : **22.01.88 FR 8800676**

(43) Date de publication de la demande :
**02.08.89 Bulletin 89/31**

(45) Mention de la délivrance du brevet :
**16.10.91 Bulletin 91/42**

(84) Etats contractants désignés :
**AT BE CH DE ES FR GB GR IT LI LU NL SE**

(56) Documents cités :
DE-A- 2 734 207
BEILSTEINS HANDBUCH DER ORGANIS-
CHEN CHEMIE, Publ. Julius Springer Verlag
Berlin, 1921-42, vol. 3, system no. 279
PATENT ABSTRACTS OF JAPAN, vol. 10, no.
31 (C-237)(2088), 6 fevr. 1986; & JP-A-60184050

(56) Documents cités :
PATENT ABSTRACTS OF JAPAN; vol. 7, NO:
151 (C-174)(1296), 2. juillet 1983; & JP-A-58
62136
PATENT ABSTRACTS OF JAPAN, vol. 10, no.
276 (C-373)(2322), 19. septembre 1986; & JP-
A-61 97247

(73) Titulaire : **SOCIETE FRANCAISE HOECHST**
Société anonyme dite:
3, avenue du Général de Gaulle
F-92800 Puteaux (FR)

(72) Inventeur : **Christidis, Yani**
53, Boulevard de la Villette
F-75019 Paris (FR)
Inventeur : **Vallejos, Jean-Claude**
9, rue Labat
F-75018 Paris (FR)

(74) Mandataire : **Rinuy, Santarelli**
14, avenue de la Grande Armée
F-75017 Paris (FR)

EP 0 326 470 B1

## Description

La présente invention concerne un procédé perfectionné de fabrication de pyruvates d'alkyle en particulier les pyruvates d'alkyle, de formule générale (I)

$$CH_3-CO-COOR \quad (I)$$

dans laquelle "R" représente un radical d'alkyle en $C_1$-$C_8$. Suivant ce procédé on obtient ces pyruvates par oxydation ménagée et sous des conditions particulières du lactate d'alkyle correspondant de formule générale (II)

$$CH_3-CHOH-COOR \quad (II)$$

dans laquelle "R" a la signification donnée précédemment.

Les produits de formule générale (I) sont des matières premières précieuses pour accéder à certaines molécules présentant d'intéressantes propriétés thérapeutiques.

Il est connu d'oxyder un lactate d'alkyle en pyruvate d'alkyle correspondant, soit en phase vapeur avec l'air ou l'oxygène en présence d'un catalyseur métallique tel que le platine, l'argent, le vanadium, éventuellement déposé sur alumine à des températures comprises entre 190°C et 500°C, soit en phase liquide par le permanganate de potassium seul ou en mélange avec du sulfate de cuivre II (cf Beilsteins Handbuch der Organischen Chemie, Vol. III, 4ème supplément page 1513).

Selon Beilsteins Handbuch Der Organischen Chemie, Vol. 3, page 608, 2ème supplément page 395 et 3ème supplément page 1148, il est également connu d'accéder à l'acide pyruvique par oxydation, soit de l'acide lactique par le réactif de Fenton (H.S.H.) Fenton et al, J. Chem. Soc. 1900, 77, 78) ou par l'oxygène en présence de pyrophosphate de fer II en solution aqueuse tamponnée (H.A. SPOEHR et al, J Amer. Chem. Soc. 1934, 56, 2070) soit par oxydation de la thymine avec le réactif de Fenton (O. BAUDISCH et al, J. Amer. Chem. Soc. 1924, 46, 188), soit encore par oxydation de l'acide lactique par l'eau de brome en présence de lumière solaire (CIUSA et al, R.A.L. [5] 23I, 821 ; Gazz. Chim. Ital. 45I, 59).

Toutefois, ces procédés connus ne donnent pas entière satisfaction. En particulier, les réactions d'oxydation catalytiques quoique sélectives sont rarement complètes et les réactions d'oxydation par voie chimique quoique complètes sont, soit peu sélectives, soit fournissent des quantités importantes de sels minéraux.

En conséquence, en fin de réaction, l'isolement du produit cherché est laborieux et onéreux d'autant plus qu'il est difficile de séparer par distillation le lactate d'alkyle non transformé du pyruvate d'alkyle correspondant étant donné la proximité de leur point d'ébullition.

Le procédé selon l'invention de fabrication de pyruvates d'alkyle de formule générale (I) par oxydation ménagée du lactate d'alkyle correspondant remédie à ces inconvénients.

Il se caractérise par le fait que cette oxydation ménagée est réalisée avec une solution aqueuse de péroxyde d'hydrogène en présence de quantités catalytiques de brome.

La solution aqueuse de peroxyde d'hydrogène peut être choisie de façon non limitative parmi les différentes solutions aqueuses de peroxyde d'hydrogène commerciales, mais, avantageusement, on utilise une solution aqueuse de peroxyde d'hydrogène de 30 à 70% en poids et préférentiellement une solution aqueuse de peroxyde d'hydrogène à 50% en poids.

Le rapport molaire du peroxyde d'hydrogène au lactate d'alkyle peut être compris entre 1 et 1,2 mais, préférentiellement il est égal à 1.

Le brome est utilisé en quantités catalytiques, de l'ordre de 10 à 200 mmoles par mole de lactate d'alkyle mis en oeuvre. Avantageusement, on utilise 50 à 200 mmoles de brome par mole de lactate d'alkyle et préférentiellement 100 mmoles.

Le procédé est réalisé au sein d'un solvant organique, non miscible à l'eau, inerte vis à vis des réactifs utilisés et dans lequel le lactate d'alkyle mis en oeuvre est soluble, tel que le dichlorométhane, le trichloro-1,1,1,éthane, le chlorobenzène. Avantageusement, on utilise le dichlorométhane.

La température réactionnelle est avantageusement comprise entre 15°C et 30°C et, préférentiellement, elle est voisine de 25°C. La réaction d'oxydation étant exothermique, il est nécessaire — d'une part — d'introduire lentement le peroxyde d'hydrogène dans le milieu réactionnel et — d'autre part — de refroidir le milieu réactionnel pour maintenir sa température dans les limites indiquées précédemment.

La réaction d'oxydation est aisément suivie par l'analyse chromatographique en phase gazeuse de prises d'essai prélevées périodiquement.

Au cours de la réaction d'oxydation, il peut se former de petites quantités de l'hydroperoxy-2-hydroxy-2 propanoate d'alkyle correspondant, facilement dégradé en pyruvate d'alkyle correspondant, soit par chauffage, soit préférentiellement par traitement avec un agent réducteur adéquat tel que le sulfite de sodium.

En fin de réaction, on isole le pyruvate d'alkyle cherché par des moyens connus en soi. Avantageusement, on neutralise d'abord le milieu réactionnel biphasique avec de l'hydrogènocarbonate de sodium, puis, après décantation, on traite la phase organique avec une solution aqueuse de sulfite de sodium et enfin, après élimination du solvant réactionnel, on isole le pyruvate d'alkyle cherché par distillation sous pression réduite.

Selon une variante, le procédé de la présente invention est effectué sous l'influence d'un rayonnement visible ou proche ultraviolet de longueurs d'ondes comprises entre environ 250 nm et 700 nm. Avantageusement, on réalise le procédé de la présente invention en présence d'un rayonnement visible fournit par une ou plusieurs lampes à incandescence classiques. Le rayonnement a pour effet d'accélérer notablement la vitesse de la réaction sans toutefois modifier le rendement final.

Les exemples suivants illustrent l'invention sans toutefois la limiter.

## EXEMPLE 1

Dans un tricol en verre de 4 litres, équipé d'un thermomètre, d'un système d'agitation mécanique et irradié par une lampe à incandescence de 200 watts, on dissout à la température ambiante dans 2660 g de dichlorométhane :
- 472 g (4 moles) de lactate d'éthyle,
- 64 g (0,4 mole) de brome.

Puis on introduit dans cette solution irradiée, en 90 minutes, sous agitation, et en maintenant la température du milieu réactionnel à 25°C par un léger refroidissement extérieur :
- 272 g de peroxyde d'hydrogène en solution aqueuse à 50% en poids, soit 4 moles.

On abandonne ensuite le milieu réactionnel toujours sous irradiation à 25°C et sous agitation.

Après 3 heures, l'analyse d'une prise d'essai par chromatographie en phase vapeur révèle la disparition complète du lactate d'éthyle. Le milieu réactionnel est alors neutralisé sous agitation avec de l'hydrogènocarbonate de sodium puis il est décanté. La phase organique après lavage avec une solution aqueuse de 50,4 g de sulfite de sodium dans 500 g d'eau est ensuite concentrée, puis l'huile résiduelle est distillée sous 80 mbars. On isole ainsi 372 g (3,2 moles) de pyruvate d'éthyle, soit un rendement de 80,2% de la théorie calculée par rapport au lactate d'éthyle.

## EXEMPLE 2

En reproduisant l'exemple 1 au départ du lactate de méthyle, on isole du pyruvate de méthyle avec un rendement de 72%.

## EXEMPLE 3

En reproduisant l'exemple 1 au départ du lactate de butyle, on isole du pyruvate de butyle pur avec un rendement de 88%.

## EXEMPLE 4

On reproduit l'exemple 1 à la lumière du jour, en l'absence du rayonnement fourni par la lampe à incandescence. La disparition complète du lactate d'éthyle demande 6 heures après la fin de l'introduction du peroxyde d'hydrogène. Après traitement, on isole le pyruvate d'éthyle cherché avec un rendement de 80% de la théorie.

## EXEMPLE 5

On reproduit l'exemple 1 dans l'obscurité complète, puis on abandonne le milieu réactionnel pendant 10 heures sous agitation, à 25°C et dans l'obscurité. Après traitement, on isole le pyruvate d'éthyle cherché avec un rendement de 80% de la théorie.

Il va du reste de soi que la présente invention n'a été décrite qu'à titre purement explicatif et nullement limitatif et que toute modification utile pourra y être apportée au niveau des équivalences sans sortir de son cadre.

## Revendications

1. Perfectionnement au procédé de fabrication de pyruvates d'alkyle de formule générale (I)

$$CH_3\text{-}CO\text{-}COOR \quad (I)$$

dans laquelle "R" représente un radical alkyle en $C_1$-$C_8$, par oxydation ménagée du lactate d'alkyle correspondant caractérisé par le fait que cette oxydation ménagée est réalisée avec une solution aqueuse de peroxyde d'hydrogène en présence de quantités catalytiques de brome.

2. Perfectionnement selon la revendication 1, caractérisé par le fait que le rapport molaire du peroxyde d'hydrogène au lactate d'alkyle est compris entre 1 et 1,2.

3. Perfectionnement selon l'une quelconque des revendications 1 et 2, caractérisé par le fait que la solution aqueuse de peroxyde d'hydrogène contient de 30 à 70% en poids de peroxyde d'hydrogène.

4. Perfectionnement selon l'une quelconque des revendications 1 à 3, caractérisé par le fait que les quantités catalytiques de brome sont comprises entre 10 et 200 mmoles par mole de lactate d'alkyle mis en oeuvre.

5. Perfectionnement selon l'une quelconque des revendications 1 à 4, caractérisé par le fait qu'il est réalisé au sein du dichlorométhane.

6. Perfectionnement selon l'une quelconque des revendications 1 à 5, caractérisé par le fait qu'il est réalisé en présence d'un rayonnement visible ou proche ultraviolet de longueurs d'ondes comprises entre environ 250 nm et 700 nm.

**Patentansprüche**

1. Verbesserung des Verfahrens zur Herstellung von Alkylpyruvaten der allgemeinen Formel (I),

$$CH_3-CO-COOR \quad (I)$$

worin R einen $C_1$-$C_8$-Alkylrest bedeutet, durch schonende Oxidation des entsprechenden Alkyllactats, dadurch gekennzeichnet, daß die schonende Oxidation mit einer wässerigen Wasserstoffperoxidlösung in Gegenwart von katalytischen Mengen Brom durchgeführt wird.

2. Verbesserung gemäß Anspruch 1, dadurch gekennzeichnet, daß das Molverhältnis von Wasserstoffperoxid zu Alkyllactat zwischen 1 und 1,2 beträgt.

3. Verbesserung gemäß Anspruch 1 oder 2, dadurch gekennzeichnet, daß die wässerige Wasserstoffperoxidlösung 30 bis 70 Gew.% Wasserstoffperoxid enthält.

4. Verbesserung gemäß einem der Ansprüche 1 bis 3, dadurch gekennzeichnet, daß die katalytischen Mengen Brom zwischen 10 und 200 mmol pro Mol Ausgangsalkyllactat betragen.

5. Verbesserung gemäß einem der Ansprüche 1 bis 4, dadurch gekennzeichnet, daß sie in Dichlormethan durchgeführt wird.

6. Verbesserung gemäß einem der Ansprüche 1 bis 5, dadurch gekennzeichnet, daß sie in Gegenwart einer sichtbaren Strahlung des nahen Ultraviolett mit einer Wellenlänge im Bereich von 250 bis 700 nm durchgeführt wird.

**Claims**

1. An improved process for the preparation of alkyl pyruvates having the general Formula (I)

$$CH_3-CO-COOR \quad (I)$$

where R denotes a $C_1$-$C_8$ alkyl radical, by controlled oxidation of the corresponding alkyl lactate, characterized in that the controlled oxidation is performed using an aqueous peroxide solution in the presence of catalytic quantities of bromine.

2. An improvement according to claim 1, characterized in that the molar ratio between the hydrogen peroxide and the alkyl lactate is between 1 and 1.2.

3. An improvement according to either of claims 1 and 2, characterized in that the aqueous hydrogen peroxide solution contains 30 to 70% by weight of hydrogen peroxide.

4. An improvement according to any of claims 1 to 3, characterized in that the catalytic quantities of bromide are between 10 and 200 mmoles per mole of alkyl lactate used.

5. An improvement according to any of claims 1 to 4, characterized in that it is performed in dichloromethane.

6. An improvement according to any of claims 1 to 5, characterized in that it is performed in the presence of visible or near ultraviolet radiation of wavelengths between approximately 250 nm and 700 nm.